# EUROPEAN PATENT APPLICATION

(11) **EP 4 538 698 A1**
(43) Date of publication of application: **16.04.2025**
(21) Application number: 24206117.4
(22) Date of filing: 11.10.2024
(51) Int. Cl.: G01N 30/78, G01N 30/86, G01N 30/68, G01N 30/72, G01N 30/74

(54) **CHEMICAL ANALYSIS METHOD AND IMPLEMENTATIONS THEREOF**

(30) Priority: 11.10.2023 US 202363589461 P; 09.10.2024 US 202418910969
(71) Applicant: Separation Systems Inc., Gulf Breeze, FL 32561 (US)
(72) Inventor: LUBKOWITZ, Joachim A., Gulf Breeze, FL 3256 (US); MENEGHINI, Roberto, Gulf Breeze, FL 3256 (US)
(74) Representative: Marks & Clerk LLP

(57) **Abstract**

A computer-implemented method for determining a composition of a chemical sample via a computer which has a memory, a processor, and a display unit, the method including uploading signals from a first data source and signals from a second data source to the memory; deconvoluting, via the processor, the signals from the first data source to provide ion identity information on ions contained in the chemical sample; transferring, via the processor, the ion identity information to ion concentration information provided by the signals from the second data source; creating, via the processor, a combined record for the chemical sample; and providing an output of the combined record on the display unit.

## Description

### CROSS-REFERENCE TO RELATED APPLICATIONS

This application claims the benefit of U.S. Provisional Patent Application No. 63/589,461, filed October 11, 2023, and hereby incorporates by reference herein the contents of this application in its entirety.

### TECHNICAL FIELD

The present disclosure is directed to a method for determining a composition of a chemical sample and practical implementations of the method.

### BACKGROUND

In the art of chromatography, several different tools exists for analyzing a chemical sample. Some tools, such as the mass spectrometer, are capable of identifying constituents of a sample, whereas others such as the flame ion detector, are capable of providing information on the relative amounts of the constituents. However, particularly when the chemical sample includes potentially harmful aromatic components, it is critical to know both the identity and the relative amount of constituents of a chemical sample.

### SUMMARY

The present disclosure is directed to a method for determining a composition of a chemical sample, the method including uploading signals from a first data source and signals from a second data source to a memory, deconvoluting the signals from the first data source via a processor to provide ion identity information on ions contained in the chemical sample, transferring the ion identity information to ion concentration information provided by the signals from the second data source via the processor, creating a combined record for the chemical sample via the processor, and providing an output of the combined record on a display unit.

The present disclosure is also directed to a method for determining a composition of a chemical sample, the method including uploading signals from a second data source to a memory, transferring, via a processor, ion identity information from a first data source to ion concentration information provided by the signals from the second data source, creating, via the processor, a combined record for the chemical sample, and providing an output of the combined record on a display unit.

Also disclosed herein are implementations of the method as described herein to determine the identity and concentration of constituents of a chemical sample. Example implementations may include simultaneously analyzing a chemical sample via a first data source and a second data source prior to uploading the signals from the first data source and the signals from the second data source to the memory to create the combined record.

The present disclosure is also directed a system for determining a composition of a chemical sample, the system comprising a computer comprising a memory, a processor. The processor is configured to: upload signals from a first data source and signals from a second data source to the memory; deconvolute the signals from the first data source to provide ion identity information on ions contained in the chemical sample; transfer the ion identity information to ion concentration information provided by the signals from the second data source; create a combined record for the chemical sample; and output the combined record.

### BRIEF DESCRIPTION OF THE DRAWINGS

FIG. 1 shows example output from the method according to aspects of the present disclosure.
FIG. 2 shows example output from the method according to aspects of the present disclosure.
FIG. 3A shows an example implementation of the method according to aspects of the present disclosure.
FIG. 3B shows an example implementation of the method according to aspects of the present disclosure.
FIG. 4 presents an example system diagram of various hardware components and other features, for use in accordance with aspects of the present disclosure; and
FIG. 5 is a block diagram of various example system components, in accordance with aspects of the present disclosure.
FIG. 6A shows one example display unit view of a computer system according to aspects of the present disclosure.
FIG. 6B shows one example display unit view of a computer system according to aspects of the present disclosure.
FIG. 7 shows one example display unit view of a computer system according to aspects of the present disclosure.
FIG. 8A shows a first selectable display unit view of a computer system according to aspects of the present disclosure.
FIG. 8B shows a second selectable display unit view of a computer system according to aspects of the present disclosure.
FIG. 8C shows a third selectable display unit view of a computer system according to aspects of the present disclosure.
FIG. 9 shows one example display unit view of a computer system according to aspects of the present disclosure.

### DETAILED DESCRIPTION

The present disclosure is directed to a method for determining the composition of a chemical sample using data from at least two different data sources, including data on the identity of constituents of the chemical sample and data on the concentration of constituents of the chemical sample. According to some aspects, a combined record of the data from the at least two different data sources is generated. The at least two different data sources may include two or more of a flame ion detector, a mass spectrometer, and a vacuum ultraviolet detector that are configured to simultaneously analyze a chemical composition. Thus in some embodiments, the flame ion detector, the mass spectrometer and/or the vacuum ultraviolet detector measure first and second data in order to provide the at least two different data sources. For example, the mass spectrometer measures the first data and the flame ion detector measures the second data. In some further embodiments, the measurement of the first and second data is performed in real time. In particular embodiments, such measurement excludes simulated measurement. That is, the measurements may be real-world (e.g. not simulated) measurements of real-world (e.g. not simulated) compositions. Also discloses herein are implementations of the method to determine the identity and concentration of constituents of a chemical sample.

As used herein, the chemical sample may be a sample of any chemical composition analyzable via chromatography, particularly gas chromatography. As used herein, gas chromatography refers to a chromatography analysis in which the mobile phase is a gas.

According to some aspects, the chemical sample may include a composition that has a boiling point suitable for use with a flame ion detector, a mass spectrometer, and/or a vacuum ultraviolet detector. In some non-limiting examples, the chemical sample may have a boiling point of no greater than about 250° C, optionally no greater than about 225° C. According to some aspects, the chemical sample may include a composition that includes one or more aromatic constituents as well as other hydrocarbon types such as naphtjenes, isoparaffins, paraffins, napthenes, olefins and oxygenated compounds. In some non-limiting examples, the chemical sample may include crude oil, gasoline, and/or petroleum products.

The method of the present disclosure determines the composition of a chemical sample using data from at least two different data sources, which may include data on the identity of constituents of the chemical sample and/or data on the concentration of constituents of the chemical sample. According to some aspects, the at least two different data sources may include two or more of a flame ion detector, a mass spectrometer, and a vacuum ultraviolet detector configured to simultaneously analyze a chemical composition. As used herein, the term "simultaneous" refers to within approximately three seconds. Additionally or alternatively, at least one of the data sources may be a computer system memory having data stored therein. In some non-limiting examples, the data may include data previously collected from a flame ion detector, a mass spectrometer, and/or a vacuum ultraviolet detector as described herein (e.g., from a previous analysis of a chemical sample). Additionally or alternatively, the data may be uploaded to the memory directly (i.e., without requiring analyzing a chemical sample via a flame ion detector, a mass spectrometer, and/or a vacuum ultraviolet detector).

As known in the art, a mass spectrometer identifies chemical constituents that pass through it by breaking each compound contained in a chemical sample into ionized fragments and detecting these fragments using the mass to charge ratio of the fragments.

A flame ion detector is an ion detector that uses an air-hydrogen flame to produce ions. As constituents of a chemical sample exit a gas chromatograph, they pass through the flame and are burned, producing ions. The ions then produce an electric current, which is used to provide the signal output of the flame ion detector.

A vacuum ultraviolet detector utilizes high energy, short wavelength photons to probe electronic transitions in almost all chemical bonds, including ground state to excited state, to identify individual compounds contained in a chemical sample.

In some non-limiting examples, the flame ion detector, mass spectrometer, and/or vacuum ultraviolet detector of the present disclosure may be provided as part of a single system. For example. U.S. Patent No. 8,413,484 (the contents of which are expressly incorporated herein in their entirety by reference) describes a Gas Chromatography-Flame Ion Detector /Mass Spectrometry apparatus (GC-FID/MS). The apparatus includes a gas chromatograph having an injector and a column, wherein the injector is configured to insert a sample along with a flowing gas into the column. The column is in communication with a divider configured to divide constituents of the chemical sample that are exiting the column for delivery to a flame ion detector and a mass spectrometer. As described therein, the divider is configured to prevent molecular discrimination within the constituents of the chemical sample. In this way, the constituents exiting the column are analyzable by both the flame ion detector and the mass spectrometer simultaneously.

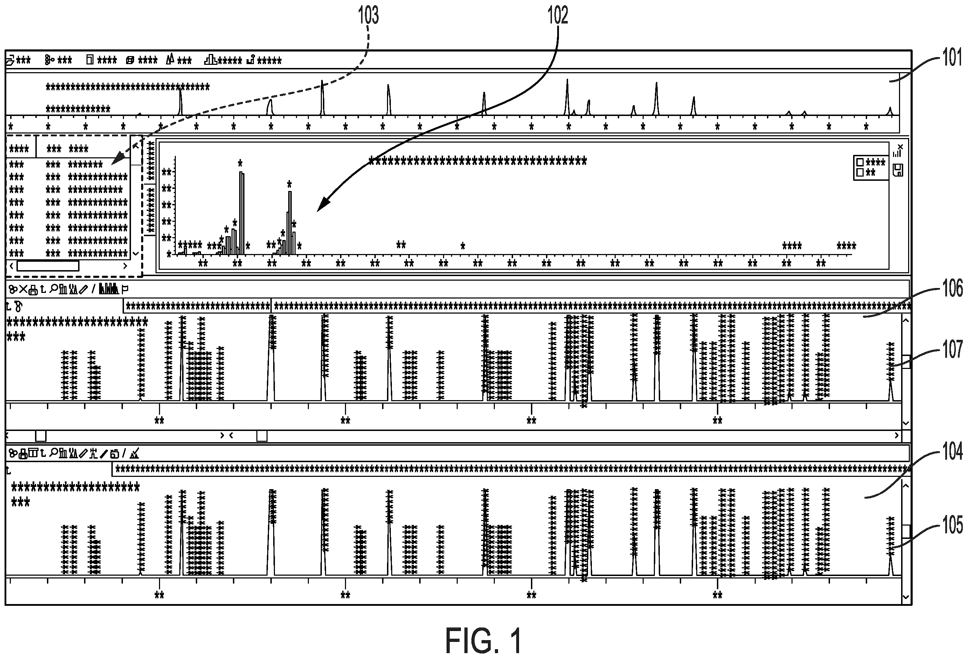

It should be understood that each of the flame ion detector, mass spectrometer, and/or vacuum ultraviolet detector will produce data that may be presented in the form of a chromatogram. For example, FIG. 1 shows an example of the data provided by a flame ion detector and a mass spectrometer as described herein. In particular, FIG. 1 shows a total ion chromatograph 101, or TIC, provided by a mass spectrometer. As known in the art, a TIC is a chromatogram that shows the summation of all mass spectral peaks from a scan of a chemical sample. Each peak in the TIC corresponds with a constituent of the chemical sample. It should be understood that all chromatograms as described herein will include legible numerical identifiers along the x- and/or y- axes as known in the art, which are represented in the drawings with asterisks. It should also be further understood that in all drawings described herein, asterisks represent legible letters, numbers, and/or symbols.

As shown in FIG. 1, the method of the present disclosure allows user to view a unique spectrum 102 corresponding to each peak of TIC 101 (and thus, each constituent of the sample). Again, this spectrum will include legible numerical identifiers along the x- and/or y- axes as known in the art, which are represented in the drawings with asterisks. Each peak of spectrum 102 corresponds with an ion contained by the constituent. In addition to spectrum 102, the method provides a user with the identity of each ion contained within each constituent, viewable in pane 103. It should be understood that information in pane 103 will be in the form of legible text and/or numbers, which are represented in the drawings with asterisks. In some non-limiting examples, the method may allow data from pane 103, i.e., ion identity, to be overlayed with peaks in spectrum 102 for easy visualization.

It should be understood that mass spectrometer data generally do not provide reliable information with regard to the concentration of constituents (i.e., ions) contained in a chemical sample. That is, TIC 101 and spectrum 102 may provide the identity of each constituent but may not provide information with regard to amounts of each constituent detected in the chemical sample.

FIG. 1 further shows an FID chromatogram 104 provided by a flame ion detector. It should be understood that FID chromatograms include peaks that each correspond with an ion contained in a chemical sample. As known in the art, the intensity of each peak of an FID chromatogram corresponds with the relative concentration of the corresponding ion in the chemical sample. In this way, FID chromatograms provide information with regard to the relative amounts of ions within a chemical sample. Unlike data provided by a mass spectrometer, however, an FID chromatogram generally does not provide an identity of ions represented by each peak.

The method of the present disclosure advantageously generates a combined record of the data from the at least two different data sources. The combined record may include data coinciding with the same time domain from each of the at least two different data sources. For example, unlike FID chromatograms known in the art, FID chromatogram 104 of FIG. 1 includes peak identifiers 105 for each peak. It should understood that peak identifier as described herein include legible text and/or numbers, which are represented in the drawings with asterisks. Peak identifiers 105 are assigned to each peak using data from TIC 101, which represents data from a mass spectrometer that is configured to analyze a chemical sample simultaneously with a flame ion detector analyzing the same chemical sample, as disclosed herein.

FIG. 1 also shows a reference chromatogram 106. According to some aspects, reference chromatogram 106 may show data stored in a memory of a computer system. Such data may be, for example, ion identify information as described herein. In this example, reference chromatogram 106 is an FID chromatogram having peak identifiers 107 as described herein, peak identifiers 107 being generated from data stored in the memory. It should be understood that at least a portion of the data shown in reference chromatograph 106 (e.g., peak identifiers 107) are not provided by a chemical analysis of a chemical sample per se. Rather, peak identifiers 107 of reference chromatograph 106 are from data stored in the memory. It should be understood that in some examples, data stored in the computer system's memory may be the first data source as described herein. In this example, a user may generate a combined record using only one of a flame ion detector, a mass spectrometer, and/or a vacuum ultraviolet detector.

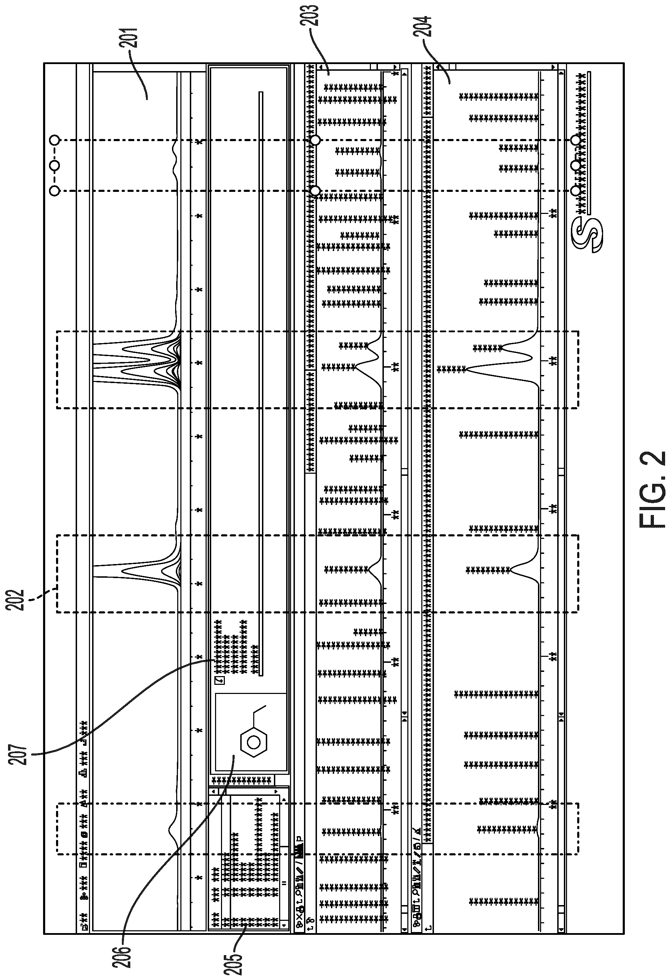

FIG. 2 shows another view of a TIC 201 and an FID chromatogram 204, similar to that of FIG. 1. As described herein, TIC 201 and FID chromatogram 204 are generated simultaneously. The method of the present disclosure thus provides a grouping 202 of peaks such that data from TIC 201 may be accurately applied to FID chromatogram 204, and vice versa. It should be understood that each grouping 202 of peaks correspond with the same time domain. In this way, a user is able to visualize, on a single record, the identity and relative concentration of each constituent identified in a chemical sample. FIG. 2 further shows a reference chromatogram 203 similar to reference chromatogram 106 as shown in FIG. 1 and a pane 205 similar to pane 103 as shown in FIG. 1. FIG. 2 also shows a pane 206 which provides structural information for a selected constituent and a pane 207 which provides further information for a selected constituent, such as a CAS Number, a chemical formula, a molecular weight, similes, or a combination thereof. It should be understood that this information may be provided as legible text and/or numbers, which are represented in the drawings with asterisks.

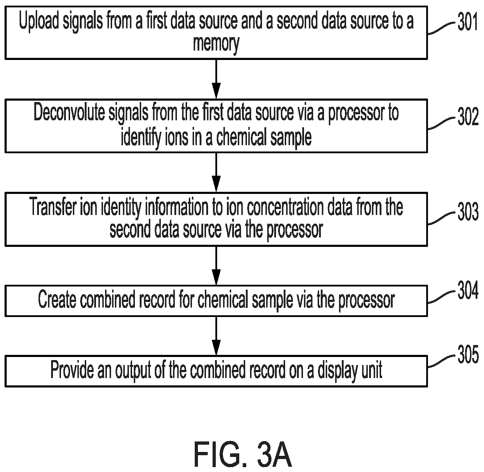

FIG. 3A shows an example process performed by the method of the present disclosure.

As shown in FIG. 3A, the process may include uploading, at 301, signals from a first data source and a second data source to a memory of a computer. The first data source may include, for example, a mass spectrometer, and the second data source may include, for example, a flame ion detector that has been configured to analyze a chemical sample simultaneously with the first data source. The process may further including deconvoluting signals from the first data source via a processor to identify ions in a chemical sample 302. For example, a TIC may be generated from mass spectrometer data as described herein. The process may further include transferring ion identity information to ion concentration data from the second data source 303, such as an FID chromatogram, via the processor. The process may further include creating a combined record for the chemical sample 304 via the processor and providing an output of the combined record on a display unit 305. In some non-limiting examples, the combined record may include an FID chromatogram with peak identifiers as described herein.

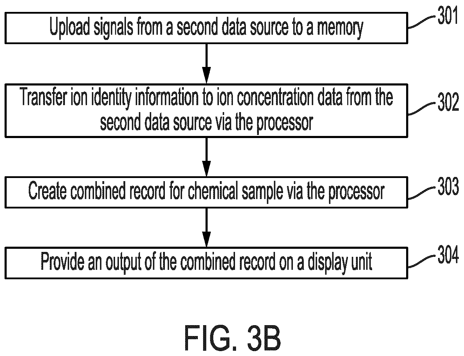

FIG. 3B shows an example process performed by the method of the present disclosure.

Similarly to the process shown in FIG. 3A, the process shown in FIG. 3B may include uploading, at 301, signals from a second data source to a memory of a computer. The second data source may include, for example, a flame ion detector that has been configured to analyze a chemical sample. The process may further include transferring ion identity information to ion concentration data from the second data source 302, such as an FID chromatogram, via the processor. As described herein, the ion identity information may be provided by a first data source, the first data source being a memory. That is, ion identity information may be provided by data already stored in the memory. The process may further include creating a combined record for the chemical sample 303 via the processor and providing an output of the combined record on a display unit 304. In some non-limiting examples, the combined record may include an FID chromatogram with peak identifiers as described herein.

The method may further include simultaneously analyzing a chemical sample via a first data source and a second data source prior to uploading signals from the first data source and the second data source to the memory.

Aspects of the present disclosure may be implemented using hardware, software, or a combination thereof and may be implemented in one or more computer systems or other processing systems. In one aspect, the disclosure is directed toward one or more computer systems capable of carrying out the functionality described herein.

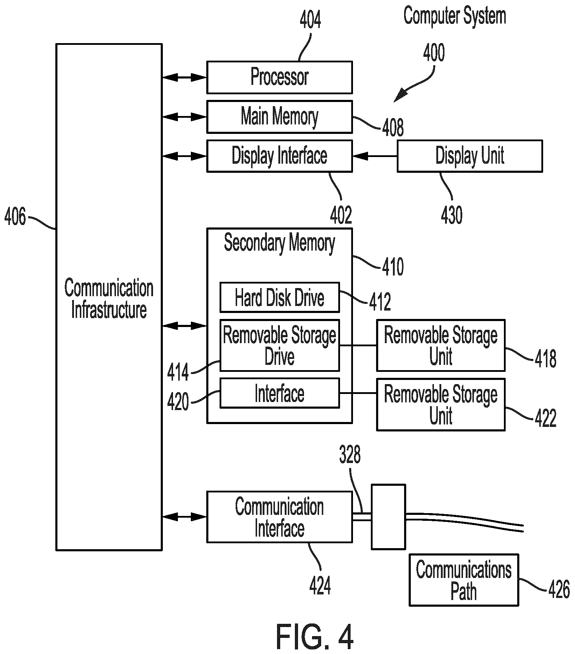

FIG. 4 presents an example system diagram of various hardware components and other features, for use in accordance with an aspect of the present disclosure. Aspects of the present disclosure may be implemented using hardware, software, or a combination thereof and may be implemented in one or more computer systems or other processing systems. In one exemplary variation, aspects of the disclosure are directed toward one or more computer systems capable of carrying out the functionality described herein. An example of such a computer system 400 is shown in FIG. 4.

Computer system 400 includes one or more processors, such as processor 404. The processor 404 is connected to a communication infrastructure 406 (e.g., a communications bus, cross-over bar, or network). Various software aspects are described in terms of this example computer system. After reading this description, it will become apparent to a person skilled in the relevant art(s) how to implement aspects of the disclosure using other computer systems and/or architectures.

Computer system 400 may include a display interface 402 that forwards graphics, text, and other data from the communication infrastructure 406 (or from a frame buffer not shown) for display on a display unit 430. Computer system 400 also includes a main memory 408, preferably random access memory (RAM), and may also include a secondary memory 410. The secondary memory 410 may include, for example, a hard disk drive 412 and/or a removable storage drive 414, representing a floppy disk drive, a magnetic tape drive, an optical disk drive, etc. The removable storage drive 414 reads from and/or writes to a removable storage unit 418 in a well-known manner. Removable storage unit 418, represents a floppy disk, magnetic tape, optical disk, etc., which is read by and written to removable storage drive 414. As will be appreciated, the removable storage unit 418 includes a computer usable storage medium having stored therein computer software and/or data.

In alternative aspects, secondary memory 410 may include other similar devices for allowing computer programs or other instructions to be loaded into computer system 400. Such devices may include, for example, a removable storage unit 422 and an interface 420. Examples of such may include a program cartridge and cartridge interface (such as that found in video game devices), a removable memory chip (such as an erasable programmable read only memory (EPROM), or programmable read only memory (PROM)) and associated socket, and other removable storage units 422 and interfaces 420, which allow software and data to be transferred from the removable storage unit 422 to computer system 400.

Computer system 400 may also include a communications interface 424. Communications interface 424 allows software and data to be transferred between computer system 400 and external devices. Examples of communications interface 424 may include a modem, a network interface (such as an Ethernet card), a communications port, a Personal Computer Memory Card International Association (PCMCIA) slot and card, etc. Software and data transferred via communications interface 424 are in the form of signals 1328, which may be electronic, electromagnetic, optical or other signals capable of being received by communications interface 424. These signals 1328 are provided to communications interface 424 via a communications path (e.g., channel) 426. This path 426 carries signals 1328 and may be implemented using wire or cable, fiber optics, a telephone line, a cellular link, a radio frequency (RF) link and/or other communications channels. In this document, the terms "computer program medium" and "computer usable medium" are used to refer generally to media such as a removable storage drive 1380, a hard disk installed in hard disk drive 1370, and signals 1328. These computer program products provide software to the computer system 400. Aspects of the disclosure are directed to such computer program products.

Computer programs (also referred to as computer control logic) are stored in main memory 408 and/or secondary memory 410. Computer programs may also be received via communications interface 424. Such computer programs, when executed, enable the computer system 400 to perform various features in accordance with aspects of the present disclosure, as discussed herein. In particular, the computer programs, when executed, enable the processor 410 to perform such features. Accordingly, such computer programs represent controllers of the computer system 400.

In variations where aspects of the disclosure are implemented using software, the software may be stored in a computer program product and loaded into computer system 400 using removable storage drive 414, hard drive 412, or communications interface 420. The control logic (software), when executed by the processor 404, causes the processor 404 to perform the functions in accordance with aspects of the disclosure as described herein. In another variation, aspects are implemented primarily in hardware using, for example, hardware components, such as application specific integrated circuits (ASICs). Implementation of the hardware state machine so as to perform the functions described herein will be apparent to persons skilled in the relevant art(s).

In yet another example variation, aspects of the disclosure are implemented using a combination of both hardware and software.

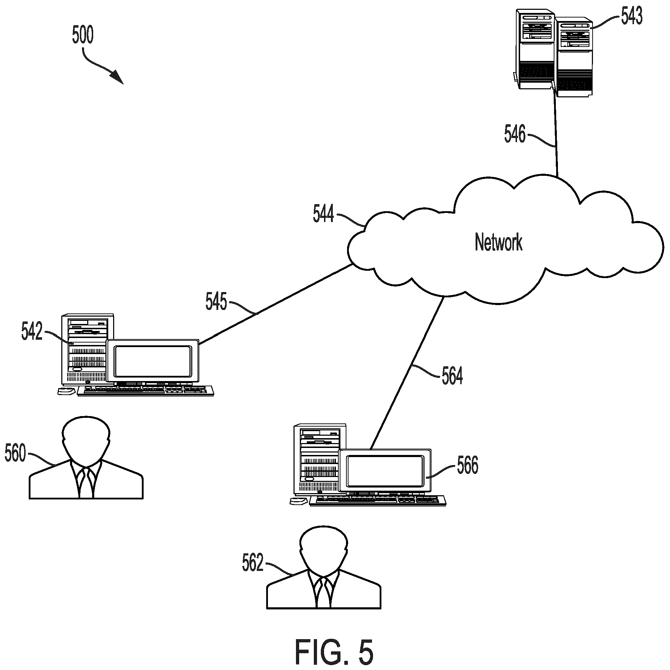

FIG. 5 is a block diagram of various example system components, in accordance with an aspect of the present disclosure. FIG. 5 shows a communication system 500 usable in accordance with the present disclosure. The communication system 500 includes one or more accessors 560, 562 (also referred to interchangeably herein as one or more "users") and one or more terminals 542, 566. In one aspect, data for use in accordance with aspects of the present disclosure is, for example, input and/or accessed by accessors 560, 562 via terminals 542, 566, such as personal computers (PCs), minicomputers, mainframe computers, microcomputers, telephonic devices, or wireless devices, such as personal digital assistants ("PDAs") or a hand-held wireless devices coupled to a server 543, such as a PC, minicomputer, mainframe computer, microcomputer, or other device having a processor and a repository for data and/or connection to a repository for data, via, for example, a network 544, such as the Internet or an intranet, and couplings 545, 546, 564. The couplings 545, 546, 564 include, for example, wired, wireless, or fiberoptic links. In another example variation, the method and system in accordance with aspects of the present disclosure operate in a stand-alone environment, such as on a single terminal.

The aspects of the disclosure discussed herein can also be described and implemented in the context of computer-readable storage medium storing computer-executable instructions. Computer-readable storage media includes computer storage media and communication media. For example, flash memory drives, digital versatile discs (DVDs), compact discs (CDs), floppy disks, and tape cassettes. Computer-readable storage media can include volatile and nonvolatile, removable and non-removable media implemented in any method or technology for storage of information such as computer readable instructions, data structures, modules or other data.

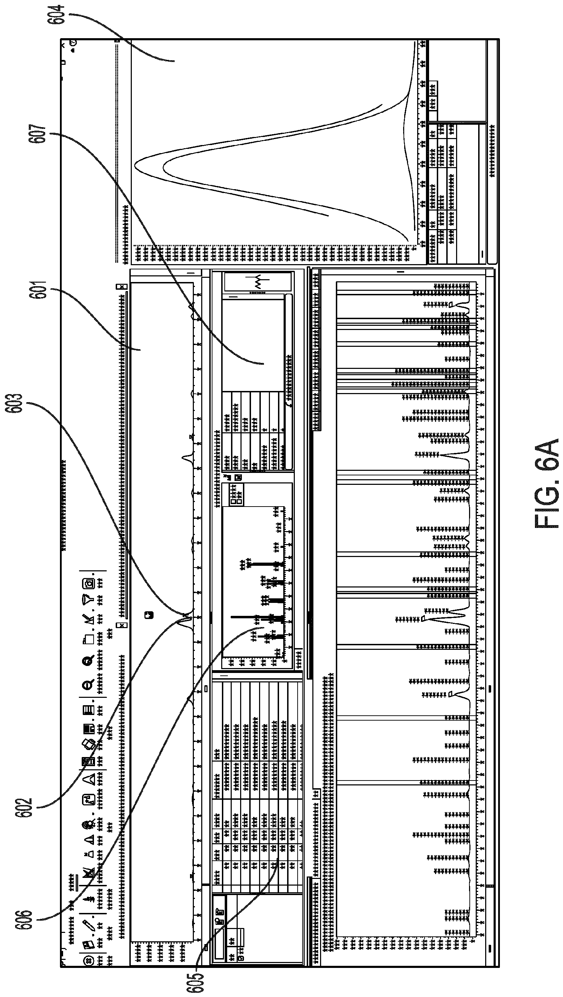

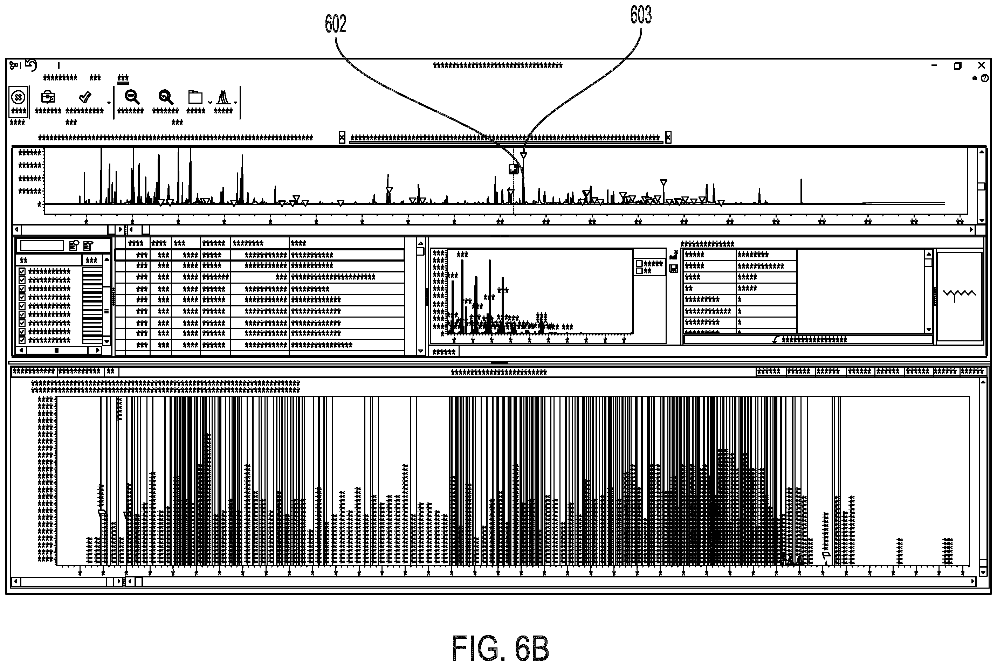

According to some aspects, the computer system of the present disclosure may automatically detect potential co-eluting components, that is, constituents of a chemical sample that are exiting (i.e., eluting from) a column simultaneously. For example, FIG. 6A shows one example display unit view of a computer system according to the present disclosure. Specifically, FIG. 6A shows a TIC 601 as described herein, TIC 601 having at least one peak 602 marked by a deconvolution symbol 603, such as a certain shape. The computer system may be such that when a user selects deconvolution symbol 603, a deconvolution display 604 may be visible, deconvolution display 604 showing deconvoluted components corresponding with peak 602. FIG. 6B shows another display unit view of a computer system according to the present disclosure. Similar to FIG. 6A, FIG. 6B shows multiple peaks 602 marked by deconvolution symbols 602.

It should be understood that similar to FIGS. 1 and 2, FIG. 6A may further including one or more of panes 605, 606, and 607, in which additional information may be provided, such as the name, composition, structure, CAS Number, chemical formula, molecular weight, and/or similes of a selected ion and/or constituent. This information may further include an estimated probability for the accuracy thereof. Again, it should be understood that this information may be provided as legible text and/or numbers, which are represented in the drawings with asterisks.

Additionally or alternatively, the computer system may automatically synchronize signals from the at least two different data sources (e.g., the flame ion detector and the mass spectrometer).

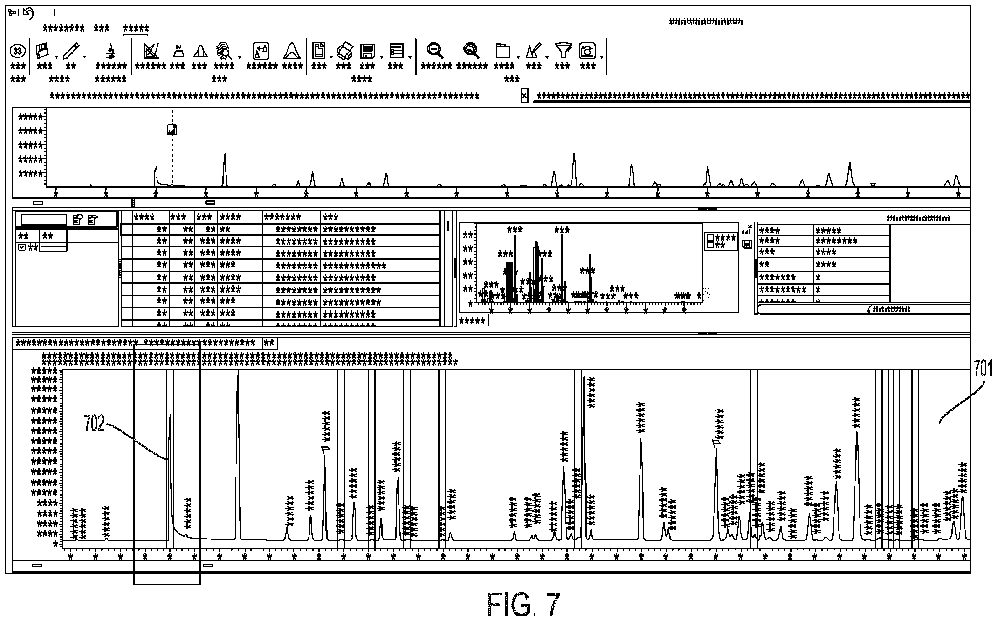

The computer system of the present disclosure may automatically extract spectral data from a selected peak of an FID chromatogram as described herein. According to some aspects, the computer system may automatically verify peak identifiers as described herein. For example, FIG. 7 shows another example display unit view of a computer system according to the present disclosure. In particular, FIG. 7 shows an FID chromatogram 701 having an identified peak 702, identified peak 702 being a peak of FID chromatogram 701 that does not match the identification provided by a first data source as described herein, such as a mass spectrometer. Identified peak 702 may be identifiable by, for example, a shape, a color, a pattern, a shading, or any combination thereof. The computer system may allow a user to define an ion-extraction series. According to some aspects, the computer system may allow for loading and/or storage of information such as the retention index deviation for a selected ion in a flame ion detector channel. Additionally or alternatively, the computer system may allow for loading and/or long-term storage of data from the at least two different data sources. For example, in the case wherein the process includes uploading signals from a first data source and/or a second data source to a memory of a computer as described herein, the computer system may allow for storage of the signals in the memory for future processes. The computer system may allow for multiple signals to be loaded simultaneously.

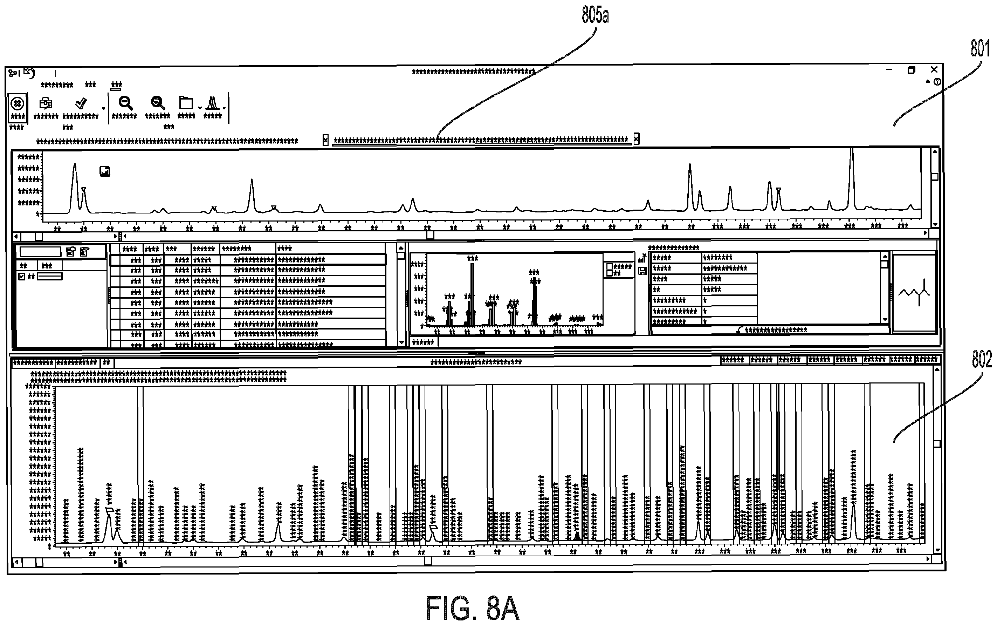

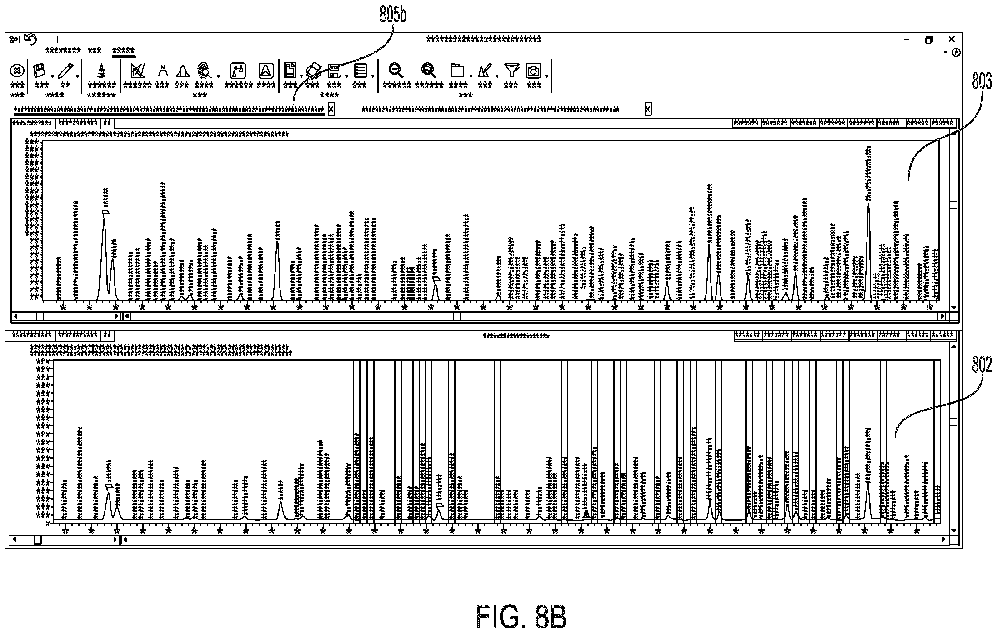

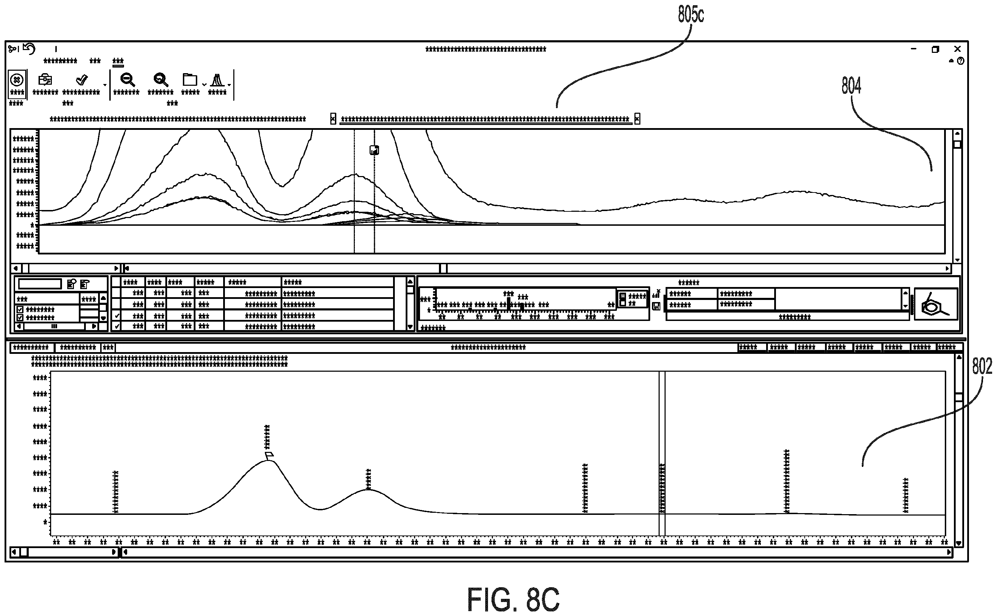

According to some aspects, the computer system may allow a user to select from two or more display unit views of a computer system of the present disclosure. For examples, FIGs. 8A-8C show three different selectable views, including a first view showing at least a TIC 801 and an FID chromatogram 802 (FIG. 8A), a second view showing FID chromatogram 802 and a reference chromatogram 803 (FIG. 8B), and a third view showing FID chromatogram 802 and a deconvolution display 604 as described herein. The first view, the second view, and the third view may be toggled by selecting one of screen selection options 808a, 805b, and 805c respectively.

According to some aspects, the memory of the computer system may include a database for storing physical properties for a plurality of chemical samples and/or constituents thereof. The database maty be storable on a memory as described herein. In some non-limiting examples, the database may include physical properties for at least presently the software has data of 8280 compounds different constituents of a chemical sample, optionally at least 2000, optionally at least 3000, optionally at least 4000, optionally at least 5000, and optionally at least 6000.

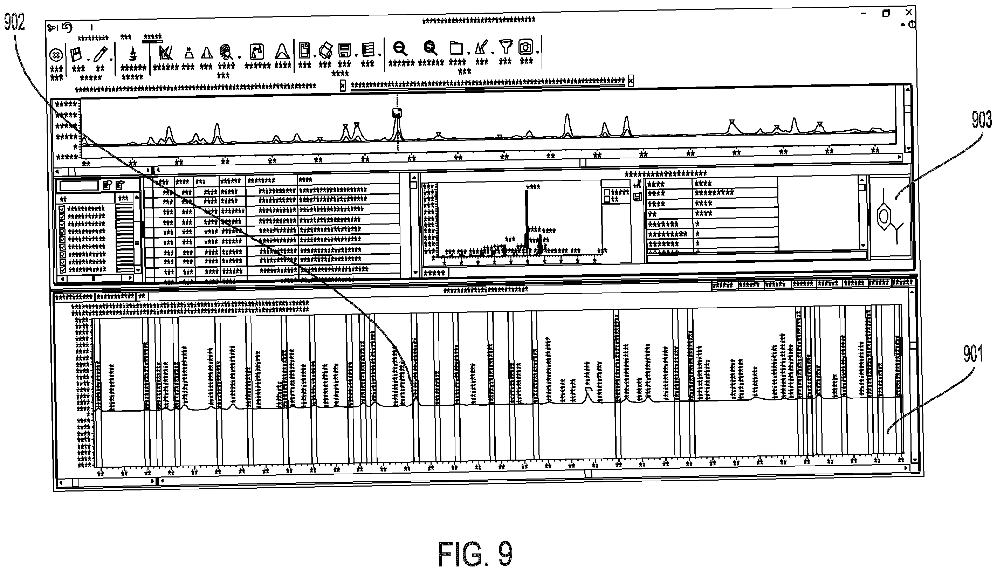

The computer system may include one or more features for physical property verification, formula editing, table editing, printing, and/or exporting data. According to some aspects, the computer system may allow a user to provide a custom output of a combined record on a display unit. For example, a user may select which peaks and/or peak identifiers to appear on a combined record as described herein. Additionally or alternatively, a user may select one or more additional data to appear on the combined record, such as average and/or standard deviations, a formula for one or more constituents, a chemical structure for one or more constituents, a retention index for one or more constituents, particulate matter index (PMI) of a chemical sample, the boiling point for one or more constituents, or a combination thereof. For example, FIG. 9 shows another example display unit view of a computer system according to the present disclosure. In particular, FIG. 9 shows an FID chromatogram 901 having a peak 902 as described herein. The computer system may be such that by selecting peak 902, a structure display 903 may become visible, structure display 903 showing the chemical structure of the constituent corresponding with peak 902. According to some aspects, a user may select a color and/or fill for each peak and/or peak identifier. According to some aspects, peaks and/or peak identifiers may be grouped and/or dragged on the display unit. The computer system may include software that detects a required driver to perform the process as described herein and/or may provide for tabulation of data. The computer system may include automatic crash recovery such that a working environment is restored to the point prior to an unmanaged shutdown.

It will be appreciated that various implementations of the above-disclosed and other features and functions, or alternatives or varieties thereof, can be desirably combined into many other different systems or applications. Also that various presently unforeseen or unanticipated alternatives, modifications, variations, or improvements therein can be subsequently made by those skilled in the art which are also intended to be encompassed by the following claims.

While the aspects described herein have been described in conjunction with the example aspects outlined above, various alternatives, modifications, variations, improvements, and/or substantial equivalents, whether known or that are or may be presently unforeseen, may become apparent to those having at least ordinary skill in the art. Accordingly, the example aspects, as set forth above, are intended to be illustrative, not limiting. Various changes may be made without departing from the spirit and scope of the disclosure. Therefore, the disclosure is intended to embrace all known or later-developed alternatives, modifications, variations, improvements, and/or substantial equivalents.

Thus, the claims are not intended to be limited to the aspects shown herein, but are to be accorded the full scope consistent with the language of the claims, wherein reference to an element in the singular is not intended to mean "one and only one" unless specifically so stated, but rather "one or more." All structural and functional equivalents to the elements of the various aspects described throughout this disclosure that are known or later come to be known to those of ordinary skill in the art are expressly incorporated herein by reference and are intended to be encompassed by the claims. Moreover, nothing disclosed herein is intended to be dedicated to the public regardless of whether such disclosure is explicitly recited in the claims. No claim element is to be construed as a means plus function unless the element is expressly recited using the phrase "means for."

Further, the word "example" is used herein to mean "serving as an example, instance, or illustration." Any aspect described herein as "example" is not necessarily to be construed as preferred or advantageous over other aspects. Unless specifically stated otherwise, the term "some" refers to one or more. Combinations such as "at least one of A, B, or C," "at least one of A, B, and C," and "A, B, C, or any combination thereof' include any combination of A, B, and/or C, and may include multiples of A, multiples of B, or multiples of C. Specifically, combinations such as "at least one of A, B, or C," "at least one of A, B, and C," and "A, B, C, or any combination thereof' may be A only, B only, C only, A and B, A and C, B and C, or A and B and C, where any such combinations may contain one or more member or members of A, B, or C. Nothing disclosed herein is intended to be dedicated to the public regardless of whether such disclosure is explicitly recited in the claims.

The examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Efforts have been made to ensure accuracy with respect to numbers used (e.g. amounts, dimensions, etc.) but some experimental errors and deviations should be accounted for.

Moreover, all references throughout this application, for example patent documents including issued or granted patents or equivalents; patent application publications; and non-patent literature documents or other source material; are hereby incorporated by reference herein in their entireties, as though individually incorporated by reference.

## Claims

1. A computer-implemented method for determining a composition of a chemical sample via a computer comprising a memory, a processor, and a display unit, the method comprising:
uploading signals from a first data source and signals from a second data source to the memory;
deconvoluting, via the processor, the signals from the first data source to provide ion identity information on ions contained in the chemical sample;
transferring, via the processor, the ion identity information to ion concentration information provided by the signals from the second data source;
creating, via the processor, a combined record for the chemical sample; and
providing an output of the combined record on the display unit.

2. The method of claim 1, wherein the first data source is a mass spectrometer.

3. The method of claim 1 or 2, wherein the second data source is a flame ion detector.

4. The method of claim 3, wherein the flame ion detector is configured to analyze the chemical sample simultaneously with the mass spectrometer.

5. The method of any one of the preceding claims, wherein the combined record comprises an FID chromatogram with peak identifiers.

6. The method of any one of the preceding claims, wherein the chemical sample has a boiling point of no greater than about 250° C.

7. The method of any one of the preceding claims, wherein the first data source and the second data source are provided as a single system.

8. The method of any one of the preceding claims, further comprising analyzing the chemical sample simultaneously via the first data source and the second data source prior to uploading the signals from the first data source and the signals from the second data source to the memory.

9. A computer-implemented method for determining a composition of a chemical sample via a computer comprising a memory, a processor, and a display unit, the method comprising:
uploading signals from a second data source to the memory;
transferring, via the processor, ion identity information from a first data source to ion concentration information provided by the signals from the second data source;
creating, via the processor, a combined record for the chemical sample; and
providing an output of the combined record on the display unit.

10. The method of claim 9, wherein the first data source is the memory.

11. A system for determining a composition of a chemical sample, the system comprising a computer comprising a memory, a processor, the processor being configured to:
upload signals from a first data source and signals from a second data source to the memory;
deconvolute the signals from the first data source to provide ion identity information on ions contained in the chemical sample;
transfer the ion identity information to ion concentration information provided by the signals from the second data source;
create a combined record for the chemical sample; and
output the combined record.

12. The system of claim 11 further comprising the first data source and the second data source, wherein the first data source and the second data source comprise one or more of:
a mass spectrometer;
a flame ion detector; and
a vacuum ultraviolet detector.

13. The system of claim 11 or claim 12 further comprising a display device, wherein the processor is configured to output the combined record to the display device.

14. The system of claim 11-13 wherein the signals from the first data source comprise a first set of measurements of a chemical sample, and wherein the signals from the second data source comprise a second set of measurements of the chemical sample.

15. The system of claim 14 configured to obtain the first set of measurements and the second set of measurements in real-time.
